# EUROPEAN PATENT APPLICATION

(11) **EP 1 958 690 A1**
(43) Date of publication of application: **20.08.2008**
(21) Application number: 08002427.6
(22) Date of filing: 11.02.2008
(51) Int. Cl.: B01J 19/00, C40B 40/06, C40B 50/18, C40B 40/10, C40B 80/00, C12Q 1/68

(54) **Substrate structure, oligomer probe array and methods for producing the same**

(30) Priority: 12.02.2007 KR 20070014534
(71) Applicant: Samsung Electronics Co., Ltd., Suwon-si, Gyeonggi-Do (KR)
(72) Inventor: Kim, Kyoung-Seon, Suwon-si, Gyeonggi-do (KR); Chi, Sung-Min, Hwaseong-si, Gyeonggi-do (KR); Hah, Jung-Hwan, Osaka Prefecture, 560-0083 (JP); Kim, Won-Sun, Yeongtong-gu, Suwon-si, Gyeonggi-do (KR)
(74) Representative: Patentanwälte Ruff, Wilhelm, Beier, Dauster & Partner

(57) **Abstract**

Disclosed are substrate structures, an oligomer probe array (160) and methods of producing the same. The substrate structure may include a substrate (110), and an intermediate film (230). The intermediate film (230) comprises either of the compounds with the following general structures: 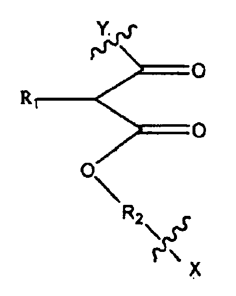 wherein R₁ is alkyl, aryl, alkoxy, nitrile, ester, phenyl, hydroxyl, aliphatic lactone, cycloalkyl or cycloalkenyl,
R₂ is alkyl, aryl, alkoxy, nitrile, ester, phenyl, hydroxyl, aliphatic lactone, cycloalkyl or cycloalkenyl, and
X is coupled with the substrate (110) directly or via an immobilization layer, Y is coupled with a linker, spacer, or an oligomer.

## Description

### BACKGROUND

### 1. Field

Example embodiments relate to substrate structures, an oligomer probe array and methods for producing the same.

### 2. Description of Related Art

As the genome project advances, genome nucleotide sequences of a variety of organisms have been found, which increased the interest in using microarrays. The microarray may be used to perform gene expression profiling and genotyping to detect mutation and polymorphism, e.g., SNP, to analyze protein and peptide, to perform screening of potential drugs, and to develop and produce new drugs.

In order to develop such an oligomer probe array, the efficiency of the molecular interface between biomaterials and semiconductors, e.g., silicon, is important to effectively and thoroughly use the intrinsic functions of the biomaterials, including the intrinsic properties of the biomaterials. For example, compounds, which allow easier binding of an oligomer probe with a substrate, and are useful as the compounds for providing a spatial margin for hybridization between the oligomer probe and a target sample, have been developed.

In the oligomer probes, for example, biomaterials, e.g., a DNA chip and/or a protein chip, immobilizing related biomaterials in a predetermined or given region on a micrometer scale may be important. The type of genetic information, ranging from genes to nucleotides, which are a minimum constituent unit of DNA, may be analyzed using the oligomer probe array. Accordingly, due to the rule of design, the probe cell may have been reduced from about tens of µm to about a few µm.

### SUMMARY

Example embodiments provide substrate structures for a highly integrated oligomer probe array having an increased reaction yield. Other example embodiments provide an oligomer probe array formed using the substrate structure. Example embodiments provide methods for producing the substrate structure and the oligomer probe array, respectively. Example embodiments are not limited to those mentioned above, and example embodiments will be apparently understood by those skilled in the art through the following description.

According to example embodiments, there is provided a substrate structure, which may include a substrate, and an intermediate film including the chemical structure represented by the following Formula 1, on the substrate. 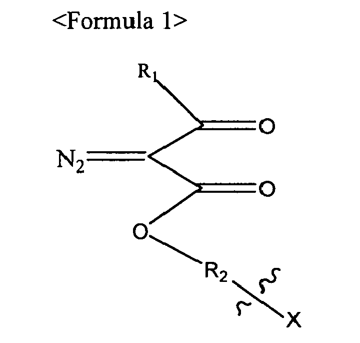
(wherein R₁ is alkyl, aryl, alkoxy, nitrile, ester, phenyl, hydroxyl, aliphatic lactone, cycloalkyl or cycloalkenyl,
R₂ is alkyl, aryl, alkoxy, nitrile, ester, phenyl, hydroxyl, aliphatic lactone, cycloalkyl or cycloalkenyl, and
X is coupled with the substrate directly or via an immobilization layer).

According to example embodiments, there is provided a substrate structure, which may include a substrate, and an intermediate film including the chemical structure represented by the following Formula 2, on the substrate. 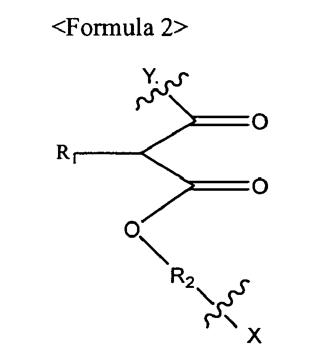
(wherein R₁ is alkyl, aryl, alkoxy, nitrile, ester, phenyl, hydroxyl, aliphatic lactone, cycloalkyl or cycloalkenyl,
R₂ is alkyl, aryl, alkoxy, nitrile, ester, phenyl, hydroxyl, aliphatic lactone, cycloalkyl or cycloalkenyl,
Y is coupled with a linker, a spacer, or an oligomer probe, and
X is coupled with the substrate directly or via an immobilization layer).

According to example embodiments, there is provided a substrate structure, which may include a substrate including an activated region coupled with an oligomer probe and a deactivated region not coupled with an oligomer probe, and an intermediate film on the substrate, including the chemical structure represented by Formula 2 on the activated region and the chemical structure represented by the following Formula 1 on the deactivated region. 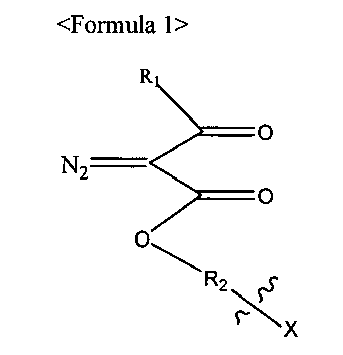 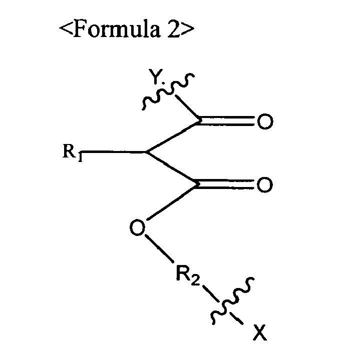
(wherein R₁ is alkyl, aryl, alkoxy, nitrile, ester, phenyl, hydroxyl, aliphatic lactone, cycloalkyl or cycloalkenyl,
R₂ is alkyl, aryl, alkoxy, nitrile, ester, phenyl, hydroxyl, aliphatic lactone, cycloalkyl or cycloalkenyl,
Y is coupled with a linker, a spacer, or an oligomer probe, and
X is coupled with the substrate directly or via an immobilization layer).

According to example embodiments, there is an oligomer probe array, which may include the substrate structure of example embodiments, and an oligomer probe on the substrate.

According to example embodiments, there is provided a method for producing the substrate structure, which may include providing a substrate, and forming an intermediate film including the chemical structure represented by the following Formula 1 on the substrate. 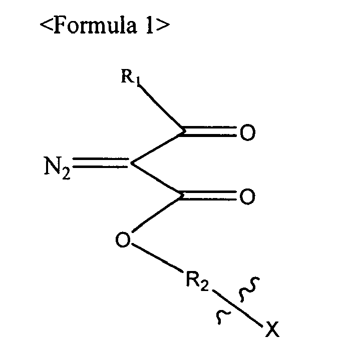
(wherein R₁ is alkyl, aryl, alkoxy, nitrile, ester, phenyl, hydroxyl, aliphatic lactone, cycloalkyl or cycloalkenyl,
R₂ is alkyl, aryl, alkoxy, nitrile, ester, phenyl, hydroxyl, aliphatic lactone, cycloalkyl or cycloalkenyl, and
X is coupled with the substrate directly or via an immobilization layer).

According to example embodiments, there is provided a method for producing the oligomer probe array, which may include producing the substrate structure according to example embodiments, exposing at least a portion of the intermediate film, and coupling at least a portion of the exposed intermediate film with an oligomer probe.

Details of other example embodiments are included in the detailed description and drawings.

### BRIEF DESCRIPTION OF THE DRAWINGS

Example embodiments will be more clearly understood from the following detailed description taken in conjunction with the accompanying drawings. FIGS. 1a-5h represent non-limiting, example embodiments as described herein.

FIGS. 1a to 1c are cross-sectional views of the microarrays according to example embodiments.

FIGS. 2a to 2d, and FIGS. 3a to 3d are cross-sectional views of the oligomer probe arrays according to example embodiments.

FIG. 4 is a sequence diagram for illustrating the method for producing the oligomer probe array according to example embodiments.

FIGS. 5a to 5h are cross-sectional views for sequentially illustrating the method for producing the substrate structure according to example embodiments.

It should be noted that these Figures are intended to illustrate the general characteristics of methods, structure and/or materials utilized in certain example embodiments and to supplement the written description provided below. These drawings are not, however, to scale and may not precisely reflect the precise structural or performance characteristics of any given embodiment, and should not be interpreted as defining or limiting the range of values or properties encompassed by example embodiments. In particular, the relative thicknesses and positioning of molecules, layers, regions and/or structural elements may be reduced or exaggerated for clarity. The use of similar or identical reference numbers in the various drawings is intended to indicate the presence of a similar or identical element or feature.

### DETAILED DESCRIPTION OF EXAMPLE EMBODIMENTS

Advantages and features of example embodiments and methods of accomplishing the same may be understood more readily by reference to the following detailed description of example embodiments and the accompanying drawings. Example embodiments may, however, be embodied in many different forms and should not be construed as being limited to the embodiments set forth herein. Rather, these embodiments are provided so that this disclosure will be thorough and complete and will fully convey the concept of example embodiments to those skilled in the art, and example embodiments will only be defined by the appended claims. In example embodiments, detailed description of known processes, device structures, and techniques incorporated herein will be omitted when it may make the subject matter of example embodiments unclear.

The terminology used herein is for the purpose of describing particular embodiments only and is not intended to be limiting of example embodiments. As used herein, the singular forms are intended to include the plural forms as well, unless the context clearly indicates otherwise. It will be further understood that the terms "comprises" and/or "comprising," when used in this specification, specify the presence of stated elements, steps, operations, and/or components, but do not preclude the presence or addition of one or more other elements, steps, operations, and/or components. Additionally, the term "and/or" includes any and all combinations of one or more of the associated listed items. Furthermore, like numbers refer to like elements throughout. Thus, the same reference numerals denote the same elements, and detailed description of such elements is omitted for the sake of convenience.

Example embodiments will be described with reference to cross-sectional views and/or schematic views, in which example embodiments are shown. Thus, the profile of an example view may be modified according to manufacturing techniques and/or allowances. For example, example embodiments are not intended to limit the scope of example embodiments but cover all changes and modifications that may be caused due to a change in the manufacturing processes. For the convenience of description, constituent elements in the drawings of example embodiments may be slightly enlarged or reduced. Example embodiments will be described in detail with reference to the accompanying drawings, hereinafter.

It will be understood that, although the terms first, second, third etc. may be used herein to describe various elements, components, regions, layers and/or sections, these elements, components, regions, layers and/or sections should not be limited by these terms. These terms are only used to distinguish one element, component, region, layer or section from another region, layer or section. Thus, a first element, component, region, layer or section discussed below could be termed a second element, component, region, layer or section without departing from the teachings of example embodiments.

Spatially relative terms, such as "beneath," "below," "lower," "above," "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is turned over, elements described as "below" or "beneath" other elements or features would then be oriented "above" the other elements or features. Thus, the exemplary term "below" can encompass both an orientation of above and below. The device may be otherwise oriented (rotated 90 degrees or at other orientations) and the spatially relative descriptors used herein interpreted accordingly.

The terminology used herein is for the purpose of describing particular embodiments only and is not intended to be limiting of example embodiments. As used herein, the singular forms "a," "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Example embodiments are described herein with reference to cross-sectional illustrations that are schematic illustrations of idealized embodiments (and intermediate structures) of example embodiments. As such, variations from the shapes of the illustrations as a result, for example, of manufacturing techniques and/or tolerances, are to be expected. Thus, example embodiments should not be construed as limited to the particular shapes of regions illustrated herein but are to include deviations in shapes that result, for example, from manufacturing. For example, an implanted region illustrated as a rectangle will, typically, have rounded or curved features and/or a gradient of implant concentration at its edges rather than a binary change from implanted to non-implanted region. Likewise, a buried region formed by implantation may result in some implantation in the region between the buried region and the surface through which the implantation takes place. Thus, the regions illustrated in the figures are schematic in nature and their shapes are not intended to illustrate the actual shape of a region of a device and are not intended to limit the scope of example embodiments.

Unless otherwise defined, all terms (including technical and scientific terms) used herein have the same meaning as commonly understood by one of ordinary skill in the art to which example embodiments belong. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

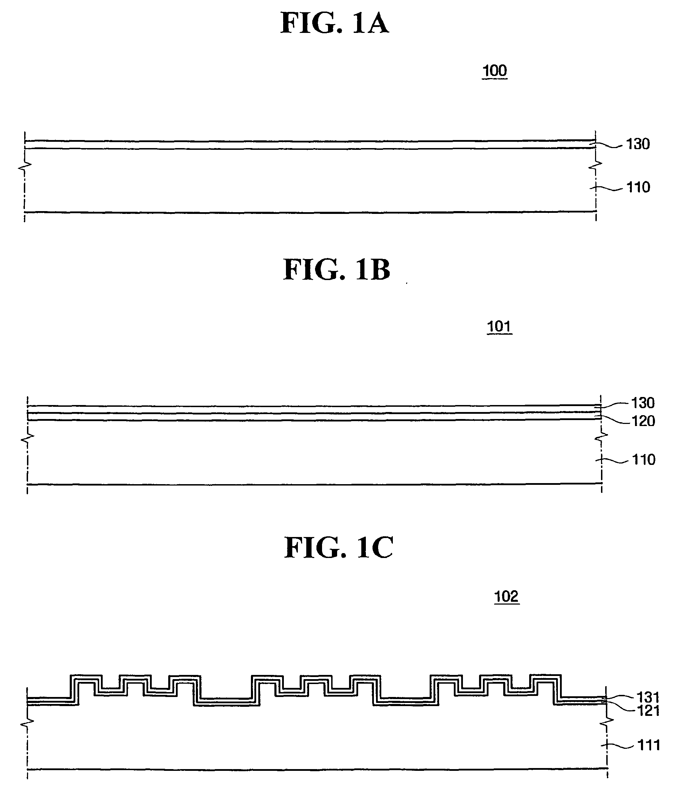

FIGS. 1a to 1c are cross-sectional views of the oligomer probe arrays according to example embodiments. With reference to FIG 1a, the substrate 100 for an oligomer probe array according to example embodiments may include a substrate 110, and an intermediate film 130 including the chemical structure represented by the following Formula 1, formed on the substrate 110. 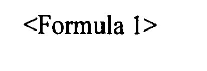 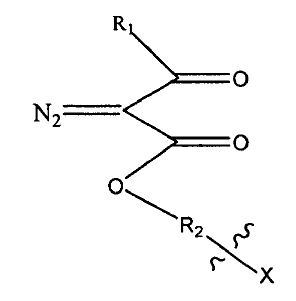
(wherein R₁ is hydroxyl, alkyl, aryl, alkoxy, nitrile, ester, phenyl, hydroxyl, aliphatic lactone, cycloalkyl or cycloalkenyl,
R₂ is alkyl, aryl, alkoxy, nitrile, ester, phenyl, hydroxyl, aliphatic lactone, cycloalkyl or cycloalkenyl,
X is coupled with the substrate directly or via an immobilization layer; and
R₁ or R₂ may be formed by subjecting a diene group selected from the compounds represented by: 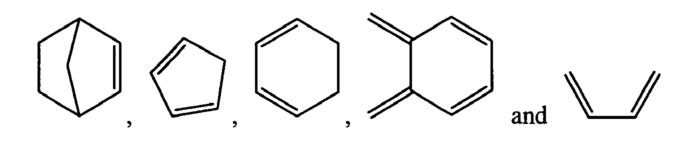 with a dienophile selected from the compounds represented by: 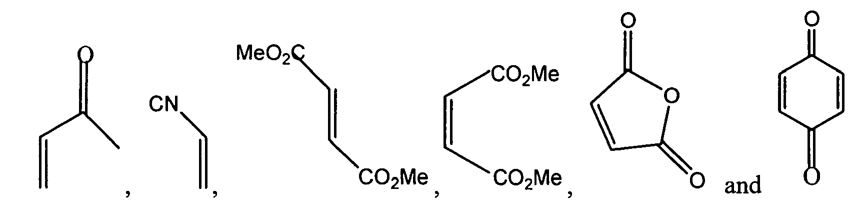 to a Diels-Alder reaction).

The substrate 110 may minimize or reduce the unwanted nonspecific bonds during a hybridization process, and further make the number of the nonspecific bonds substantially about zero. The substrate 110 may be made of materials which are transparent to visible ray and/or UV. The substrate 110 may be a flexible or rigid substrate. Examples of the flexible substrate may include membranes and/or plastic films made of nylon or nitrocellulose. Examples of the rigid substrate may include a silicon substrate, a transparent glass substrate, and/or a quartz substrate. In the case of the silicon substrate and/or the transparent glass substrate, nonspecific bonding may not occur during a hybridization process. Additionally, the transparent glass substrate may be transparent to visible ray and/or UV, thereby advantageous in detection by using fluorescent markers. The silicon substrate and/or the transparent glass substrate may be advantageous because the various processes of producing thin films or the photolithography process to produce semiconductor devices or LCD panels may be applied to them without modification.

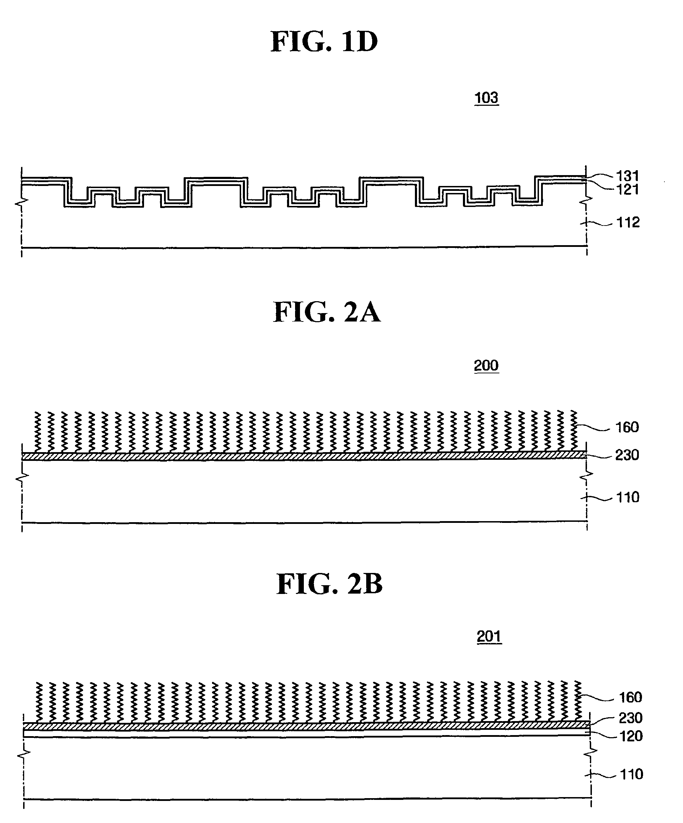

The intermediate film 130 may be formed by directly coupling X in Formula 1 with the substrate 110. The coupling means a chemical bond, for example, a covalent bond. The intermediate film 130 formed on the substrate 110 may have the chemical structure represented by Formula 1. The chemical structure represented by Formula 1 may contain a diazoketo group. If a ray of about 193 nm or about 248 nm is irradiated on the intermediate film 130 containing a diazoketo group, the diazo group may leave to form a carboxylic acid by a series of reactions. But, the intermediate film 130 illustrated in FIG 1 may contain a diazoketo group before forming the carboxylic acid.

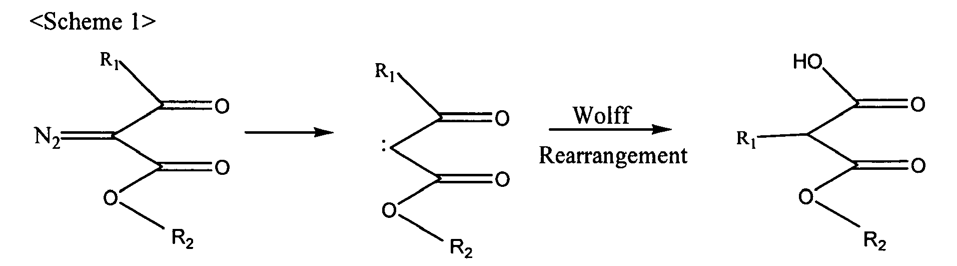

In the intermediate film 130 containing a diazoketo group, a carboxylic group may be formed by the leaving of the diazo group. As a result, the intermediate film 130 may couple with a functional group which is capable of reacting with the carboxylic group. Examples of the functional group which is capable of reacting with the carboxylic group may include an amine group and a hydroxyl group, but may not be limited thereto. Thus, the substrate 100 for an oligomer probe array may be used for an oligomer probe array by coupling with oligomer probes, linkers, spacers, the microparticles, and/or nanoparticles, which contain a functional group capable of coupling with the carboxylic group. If the intermediate film 130 is coupled with the oligomer probe, the intermediate film 130 may function as a linker or a spacer. For example, the intermediate film 130 may provide free interaction, e.g., hybridization, of the oligomer probe with a target sample, as a linker or a spacer in the oligomer probe array.

Also, in some figures, the example embodiments in which the intermediate film 130 may be formed the all surface of the substrate is exemplified, but it may be partially formed on a part of surface of the substrate 110 according to purposes. For example, the intermediate film 130 may be formed as a matrix of square shapes having regular pitch. But, the square shape is merely an example, and it may not be limited to the above shapes. Circle shapes are also possible, and can be applied equally hereinafter.

The substrate structure 101 according to example embodiments will be described in detail with reference to FIG. 1b, hereinafter. FIG. 1b is a cross-sectional view of the substrate structure according to example embodiments. The substrate structure 101, illustrated in FIG. 1b, may have basically the same structure as the substrate 100 for an oligomer probe array illustrated in FIG. 1a, except for the following. In the following embodiments, if the constituting materials of the elements as described above will be repeated again herein, description thereof shall be omitted or simplified.

The substrate structure 101 according to example embodiments may include a substrate 110, an immobilization layer 120 formed on the substrate 110, and a intermediate film 130 including the chemical structure represented by Formula 1, formed on the immobilization layer 120. In the substrate structure 101, the intermediate film 130 may be coupled with the substrate 110 via the immobilization layer 120.

The immobilization layer 120 may be a siloxane resin layer made of, for example, a siloxane resin. For example, in Formula 1, the immobilization layer may be -Si(OR)₃ (wherein R is alkyl). The presence of the immobilization layer 120 may make the coupling of the intermediate film 130 with the substrate 110 easier, and may improve the functions of the intermediate film 130 as a linker and/or a spacer, consequently increasing the reaction yield with a target sample. Here, the immobilization layer 120 can be omitted because it is used to make the coupling of the intermediate film easy.

Also, the example embodiments in which the immobilization layer 120 may be formed on all surface of the substrate is exemplified, but it may be partially formed on a part of substrate 110 according to some purposes. The manner can be substantially the same as the case of the intermediate film 130, and can be applied equally hereinafter.

The substrate structure 102 according to example embodiments will be described in detail with reference to FIG. 1c, hereinafter. FIG. 1c is a cross-sectional view of the substrate structure according to example embodiments. The substrate structure 102, illustrated in FIG. 1c, may have basically the same structure as the substrate structure 101 illustrated in FIG. 1b, except for the following.

The substrate structure 102 according to example embodiments may include a substrate 111 having a three-dimensional surface, an immobilization layer 121 formed on the substrate 111, and an intermediate film 131 including the chemical structure represented by Formula 1. The substrate structure 102 according to example embodiments may include a three-dimensional surface on the substrate 111. The intermediate film 131 may be formed on the three-dimensional surface of the substrate 111. The substrate 111, the immobilization layer 121, and the intermediate film 131 may have three-dimensional surfaces, thereby being represented by different reference numerals from the substrate 110, the immobilization layer 120, and the intermediate film 130 of the substrate 100 for an oligomer probe array according to example embodiments. However, that is the only difference, and as such, the description thereof shall also be omitted. If the substrate 111 has a three-dimensional surface, the oligomer probe may be highly integrated into the substrate structure 102, which may reduce the rule of design, and may increase the reaction yield. Below, the oligomer probe arrays according to example embodiments will be described with reference to the drawings.

Further, as shown in FIG. 1D, a substrate 103 for an oligomer probe array according to example embodiments can include a three-dimensional surface within the substrate. Further, the cross section of the three-dimensional surface has a saw-toothed shape, but it may have a three-dimensional structure. For example, the surface can include a curved surface.

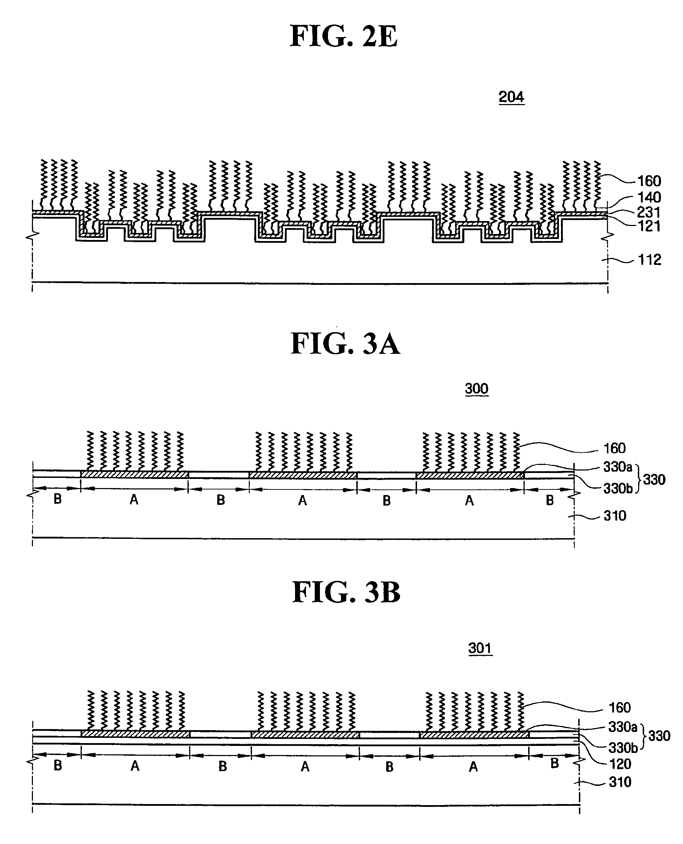

FIGS. 2a to 2d, and FIGS. 3a to 3b are cross-sectional views of the oligomer probe arrays according to example embodiments. With reference to FIG. 2a, the oligomer probe array 200 according to example embodiments may include a substrate 110, an oligomer probe 160 formed on the substrate 110, and an intermediate film 230 interposed between the substrate 110 and the oligomer probe 160, including the chemical structure represented by Formula 2. 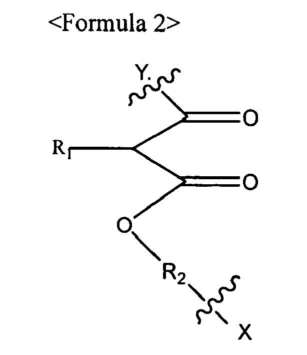
(wherein R₁ is alkyl, aryl, alkoxy, nitrile, ester, phenyl, hydroxyl, aliphatic lactone, cycloalkyl or cycloalkenyl,
R₂ is alkyl, aryl, alkoxy, nitrile, ester, phenyl, hydroxyl, aliphatic lactone, cycloalkyl or cycloalkenyl,
Y is coupled with a linker, a spacer, or an oligomer probe, and
X is coupled with the substrate directly or via an immobilization layer).

The substrate 110 may be substantially the same as the substrate structure 101 illustrated in FIG. 1b, and thus description thereof shall also be omitted. The intermediate film 230 may have the chemical structure represented by Formula 2. In the case of the substrate 100 for an oligomer probe array illustrated in FIG. 1a, the intermediate film 130 formed on the substrate 110 may have the chemical structure represented by Formula 1 containing a diazoketo group. However, the chemical structure represented by Formula 1 containing a diazoketo group may be converted into a carboxylic group upon exposure to light, and after exposure of the carboxylic group, the oligomer probe 160 will be coupled. Because the oligomer probe array 200 illustrated in FIG. 2a is coupled with the oligomer probe 160 unlike the substrate 100 for an oligomer probe array illustrated in FIG. 1a, the oligomer probe array 200 may have the chemical structure represented by Formula 2, which is due to the mechanism, as shown in Scheme 1. The intermediate film 230 may function as a linker (linker molecule) for linking the substrate 110 and the oligomer probe 160, or a spacer for providing spatial margin required for hybridization with a target sample. For example, the intermediate film 230 may be coupled with the oligomer probe to provide spatial margin required for hybridization with a target sample.

The oligomer probe 160 may refer to a polymer that is formed of two or more monomers covalently bonded to each other. The oligomer may be formed to have about 2 monomers to about 500 monomers, e.g., about 5 monomers to about 300 monomers, e.g., about 5 monomers to about 100 monomers. Examples of the monomer may include nucleosides, nucleotides, amino acids, and peptides, according to the types of the probes. Nucleosides and nucleotides may include a known purine or pyrimidine base, a methylated purine or pyrimidine, or an includacylated purine or pyrimidine. Furthermore, nucleosides and nucleotides may include known ribose or deoxyribose sugars, or may include modified sugars in which one or more hydroxyl groups are substituted by halogen atoms or aliphatics or to which the functional group, e.g., ether or amine, may be bonded.

The amino acid may be an L-, D-, and/or nonchiral-type amino acid, which is found in nature. Alternatively, the amino acid may be a modified amino acid and/or an analog of the amino acid. The peptide may refer to a compound produced by an amide bond between the carboxylic group of the amino acid and the amino group of another amino acid. The oligomer probe 160 may be made of two or more nucleosides, nucleotides, amino acids and/or peptides.

In the oligomer probe 160, the intermediate film 230 and the oligomer probe 160 may be directly coupled, and thus, the oligomer probe 160 may contain a functional group capable of coupling with a carboxylic group of the intermediate film 230. Examples of the functional group capable of coupling with a carboxylic group may include an amine group, and a hydroxyl group, but not limited thereto. The coupling may mean a covalent bond among the chemical bonds.

The oligomer probe array 201 according to example embodiments will be described with reference to FIG. 2b, hereinafter. FIG. 2b is a cross-sectional view of the oligomer probe array according to example embodiments. The oligomer probe array 201 illustrated in FIG. 2b may have basically the same structure as the oligomer probe array 200 illustrated in FIG. 2a, except for the following.

The oligomer probe array 201 according to example embodiments may include a substrate 110, an immobilization layer 120 formed on the substrate 110, an intermediate film 230 including the chemical structure represented by Formula 2, formed on the immobilization layer 120, and an oligomer probe 160 coupled with the intermediate film 230. In the oligomer probe array 201, the intermediate film 230 may be coupled with the substrate 110 via the immobilization layer 120. The immobilization layer 120 may be a siloxane resin layer made of, for example, a siloxane resin. For example, in Formula 1, the immobilization layer 120 may be -Si(OR)₃ (wherein R is alkyl). The presence of the immobilization layer 120 may make the coupling of the intermediate film 130 with the substrate 110 easier and improve the functions of the intermediate film 130 as a linker or a spacer, and consequently increasing the reaction yield with a target sample.

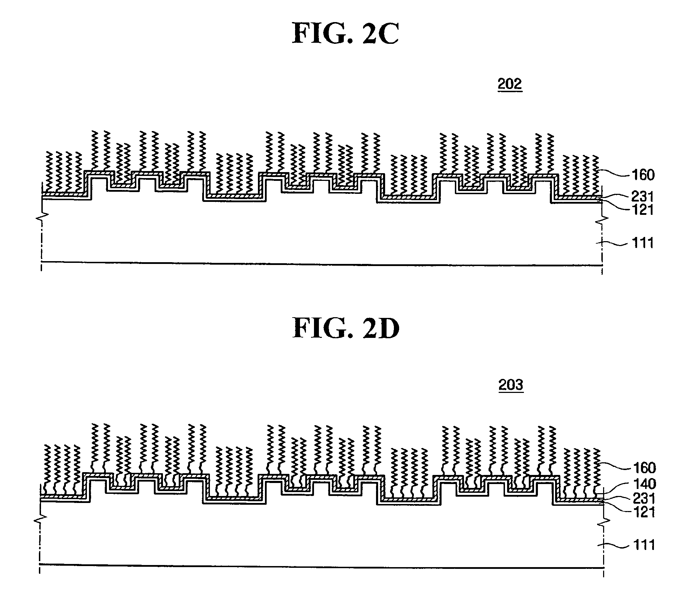

The oligomer probe array 202 according to example embodiments will be described with reference to FIG. 2c, hereinafter. FIG. 2c is a cross-sectional view of the oligomer probe array according to example embodiments. The oligomer probe array 202 illustrated in FIG. 2c may have basically the same structure as the oligomer probe array 201 illustrated in FIG. 2b, except for the following.

The oligomer probe array 202 according to example embodiments may include a substrate 111 having a three-dimensional surface, an immobilization layer 121 formed on the substrate 111, an intermediate film 231 including the chemical structure represented by Formula 2, formed on the immobilization layer 121, and an oligomer probe 160 coupled with the intermediate film 231. The substrate 111, the immobilization layer 121, and the intermediate film 231 may have three-dimensional surfaces, thereby being represented by different reference numerals from the substrate 110, the immobilization layer 120, and the intermediate film 230 of the oligomer probe array 200 illustrated in FIG 2b. However, that is the only difference, and as such, the description thereof shall also be omitted. If the substrate 111 has a three-dimensional surface, the oligomer probe 160 may be highly integrated into the oligomer probe array 202, which leads to a reduction in the rule of design and increase in the reaction yield.

The oligomer probe array 203 according to example embodiments will be described with reference to FIG. 2d, hereinafter. FIG. 2d is a cross-sectional view of the oligomer probe array according to example embodiments. The oligomer probe array 203 illustrated in FIG. 2d may have basically the same structure as the oligomer probe array 202 illustrated in FIG. 2c, except for the following.

The oligomer probe array 203 according to example embodiments may include a substrate 111 having a three-dimensional surface, an immobilization layer 121 formed on the substrate 111, an intermediate film 231 including the chemical structure represented by Formula 2, formed on the immobilization layer 121, a linker 140 coupled with the intermediate film 231, and an oligomer probe 160 coupled with the linker 140. Further, as shown in FIGS. 2E, an oligomer probe array 204 according to example embodiments may include a three-dimensional surface within the substrate 112. Further, it is sufficient to form the three-dimensional surface as a surface having a three-dimensional structure, e.g., a surface where a curved surface such as a round shape is formed.

In FIG. 2d and FIG 2E, the linker 140 is shown, but not limited thereto, and thus a spacer, microparticles, and/or nanoparticles may link the intermediate film 231 and the oligomer probe 160. The linker 140, the spacer, the microparticles, and/or the nanoparticles may contain a functional group capable of coupling with the substrate or the oligomer probe 160.

The linker 140, the spacer, the microparticles, and/or the nanoparticles may make the coupling of the oligomer probe with a substrate easier, or provide spatial margin for hybridization with a target sample. As previously discussed, the intermediate film 231 may function the same as the linker 140, the spacer, the microparticles, and/or the nanoparticles, but may also be coupled with the linker 140, the spacer, the microparticles, and/or the nanoparticles. The intermediate film 231 may function as a linker, but may also provide an oligomer probe array having an improved reaction yield by the interposition of another linker. The linker 140 may contain a functional group capable of binding with the carboxylic group of the intermediate film 231. If the oligomer probe 160 binds with an intermediate film 231, containing a functional group from binding with the carboxylic group may not be necessary.

The oligomer probe array 300 according to example embodiments will be described with reference to FIG. 3a, hereinafter. The oligomer probe array 300 illustrated in FIG. 3a may have basically the same structure as the oligomer probe array 200 illustrated in FIG. 2a, except for the following. With reference to FIG. 3a, an oligomer probe array 300 according to example embodiments may include an oligomer probe 160, a substrate 310 including an activated region A coupled with the oligomer probe 160, and a deactivated region B not coupled with the oligomer probe 160, and an intermediate film 330 including the chemical structure represented by Formula 2 on the activated region A and the chemical structure represented by the following Formula 1 on the deactivated region B. 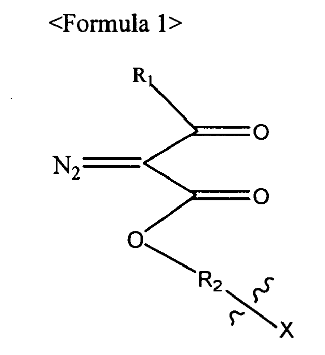 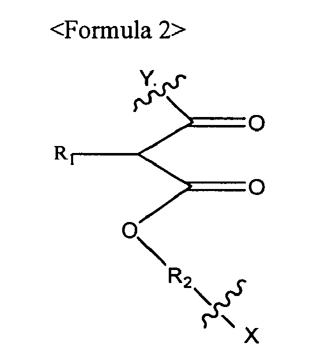
(wherein R₁ is alkyl, aryl, alkoxy, nitrile, ester, phenyl, hydroxyl, aliphatic lactone, cycloalkyl or cycloalkenyl,
R₂ is alkyl, aryl, alkoxy, nitrile, ester, phenyl, hydroxyl, aliphatic lactone, cycloalkyl or cycloalkenyl,
Y is coupled with a linker, a spacer, or an oligomer probe, and
X is coupled with the substrate directly or via an immobilization layer).

For example, the substrate 310 of the oligomer probe array 300 according to example embodiments may include an activated region A coupled with the oligomer probe 160, and a deactivated region B not coupled with the oligomer probe 160. The intermediate film 330 may include a region 330a including the chemical structure represented by Formula 2 formed on the activated region A and a region 330b including the chemical structure represented by the following Formula 1 formed on the deactivated region B. The intermediate film 330a formed on the activated region A may be coupled with the oligomer probe 160. The intermediate film 330b of the region including the chemical structure represented by the following Formula 1 formed on the deactivated region B may not be coupled with the oligomer probe 160. The oligomer probe 160 may not be coupled with the entire surface of the intermediate film 330, but a predetermined or given region 330a, thereby providing a selective activated region, which enables various oligomer probes 160 to react with a target sample more delicately. The intermediate film 330 may function as a linker (linker molecule) for linking the substrate 310 and the oligomer probe 160, or a spacer for providing spatial margin required for hybridization with a target sample.

The substrate 310 and the intermediate film 330 of the oligomer probe array 300 illustrated in FIG. 3a may be different from the substrate 110, and the intermediate film 230 of the oligomer probe array 200 illustrated in FIG. 2a, thereby being represented by different reference numerals from each other. However, they are both substantially the same except for the above described difference, and therefore, the description thereof shall also be omitted. The oligomer probe array 301 according to example embodiments will be described with reference to FIG. 3b, hereinafter. The oligomer probe array 301 illustrated in FIG. 3b has basically the same structure as the oligomer probe array 300 illustrated in FIG. 3a, except for the following.

With reference to FIG. 3b, the oligomer probe array 301 may include an oligomer probe 160, a substrate 310 including an activated region A coupled with the oligomer probe 160, and a deactivated region B not coupled with the oligomer probe 160, an immobilization layer 120 formed on the substrate 310, and an intermediate film 330 including a region 330a including the chemical structure represented by Formula 2 formed on the activated region A and a region 330b including the chemical structure represented by the following Formula 1 formed on the deactivated region B. For example, in the oligomer probe array 301 according to example embodiments, the intermediate film 330 may be coupled with the substrate 310 via an immobilization layer 120. The immobilization layer 120 may be a siloxane resin layer made of, for example, a siloxane resin. For example, in Formula 1, the immobilization layer may be -Si(OR)₃ (wherein R is alkyl). The presence of the immobilization layer 120 may make the coupling of the intermediate film 130 with the substrate 110 easier, and improve the functions of the intermediate film 130 as a linker or a spacer, consequently increasing the reaction yield with a target sample.

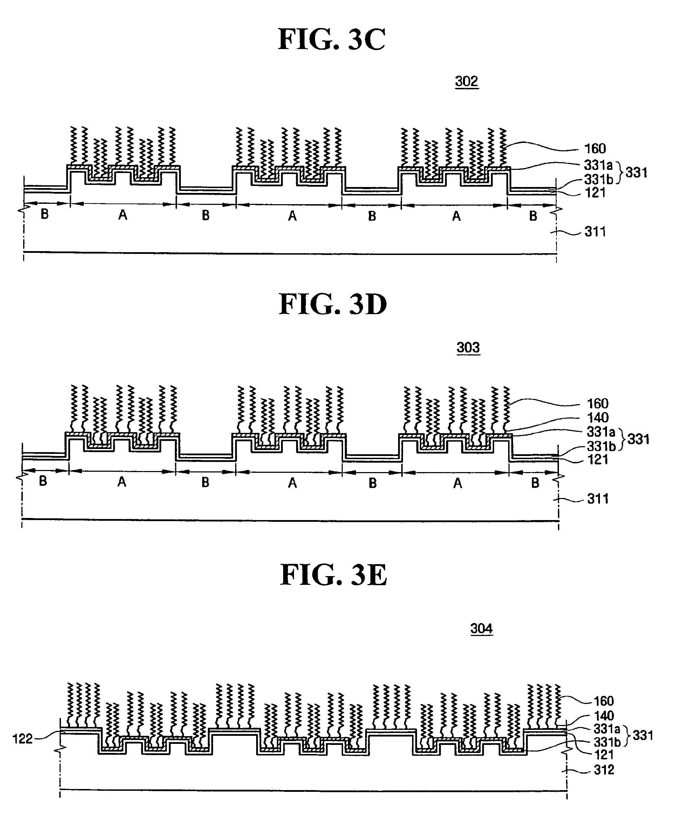

The oligomer probe array 302 according to example embodiments will be described with reference to FIG. 3c, hereinafter. The oligomer probe array 302 illustrated in FIG. 3c may have basically the same structure as the oligomer probe array 300 illustrated in FIG. 3a, except for the following. With reference to FIG. 3c, the oligomer probe array 302 illustrated in FIG. 3c may differ from the oligomer probe array 301 illustrated in FIG. 3b in that the substrate 311 may have a three-dimensional surface. Because the substrate 311 has a three-dimensional surface, the immobilization layer 121 formed on the surface of the substrate 311 and the intermediate film 331 formed on the immobilization layer 121 may also have three-dimensional surfaces, thereby being represented by different reference numerals from the intermediate film 330 of the oligomer probe array 301 illustrated in FIG. 3b. However, the oligomer probe array 302 illustrated in FIG. 3c and the oligomer probe array 301 illustrated in FIG. 3b may be substantially the same.

If the substrate 311 has a three-dimensional surface, the oligomer probe 160 may be highly integrated into the oligomer probe array 302, which may decrease the rule of design and increase the reaction yield. The oligomer probe array 303 according to example embodiments will be described with reference to FIG. 3d, hereinafter. The oligomer probe array 303 illustrated in FIG. 3d may have basically the same structure as the oligomer probe array 302 illustrated in FIG. 3c, except for the following.

With reference to FIG. 3d, the oligomer probe array 303 illustrated in FIG. 3d may differ from the oligomer probe array 302 illustrated in FIG. 3c in that the oligomer probe 160 may be coupled with the intermediate film 331 via the linker 140. In FIG. 3d, the linker 140 is shown, but not limited thereto, and thus, a spacer, the microparticles, and/or nanoparticles may link the intermediate film 331 and the oligomer probe 160. The linker 140, the spacer, the microparticles, and/or the nanoparticles may contain a functional group capable of coupling with the substrate or the oligomer probe 160, which may make the coupling of the oligomer probe with a substrate easier, or provide spatial margin for hybridization with a target sample. As discussed earlier, the intermediate film 331 may function the same as the linker 140, the spacer, the microparticles, and/or the nanoparticles, but may also be coupled with the linker 140, the spacer, the microparticles, and/or the nanoparticles, thereby providing an oligomer probe array having an improved reaction yield.

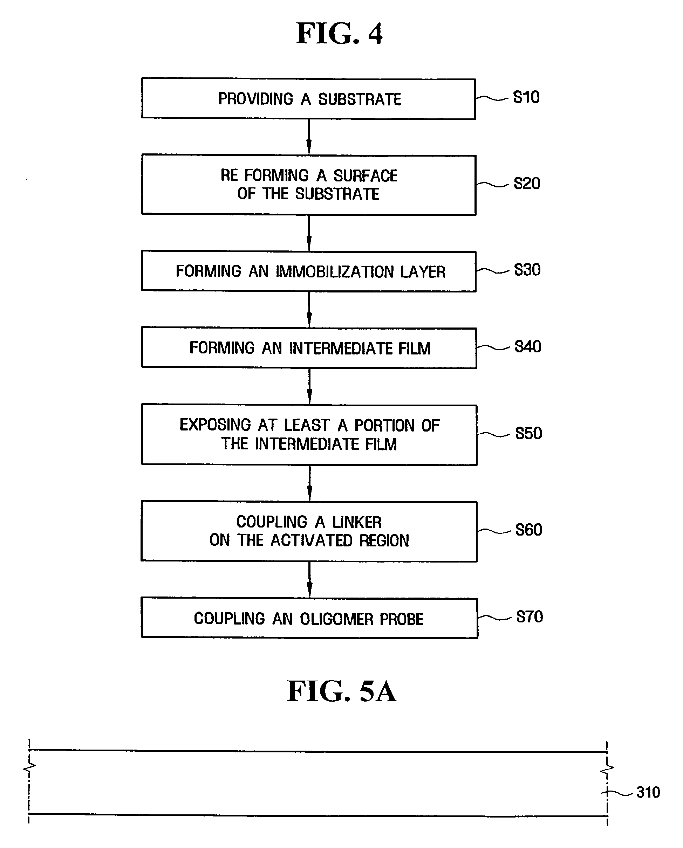

Further, as shown in FIG. 3E, an oligomer probe array 304 according to example embodiments may include a three-dimensional surface within the substrate 312. Further, the three-dimensional surface may be formed in a curved surface, and a surface having a three-dimensional structure is sufficient, as described above The method for producing the oligomer probe array according to example embodiments will be described with reference to all of the accompanying figures, hereinafter, with reference to FIG. 4, and FIGS. 5a to 5h.

FIG. 4 is a sequence diagram for describing the method for producing the oligomer probe array according to example embodiments, and FIGS. 5a to 5h are cross-sectional views for sequentially describing illustrating the method for producing the oligomer probe array according to example embodiments. As shown in FIG. 4, the method for producing the oligomer probe array 303 according to example embodiments may include providing a substrate 310 (S 10), forming a three-dimensional surface of the substrate 310 (S20), forming an immobilization layer 121 on the substrate 311 having the three-dimensional surface (S30), forming an intermediate film 331 on the immobilization layer 121 (S40), exposing at least a portion of the intermediate film 331 (S50), coupling the intermediate film 331a with a linker 140 (S60), and coupling the coupled linker 140 with an oligomer probe 160 (S70).

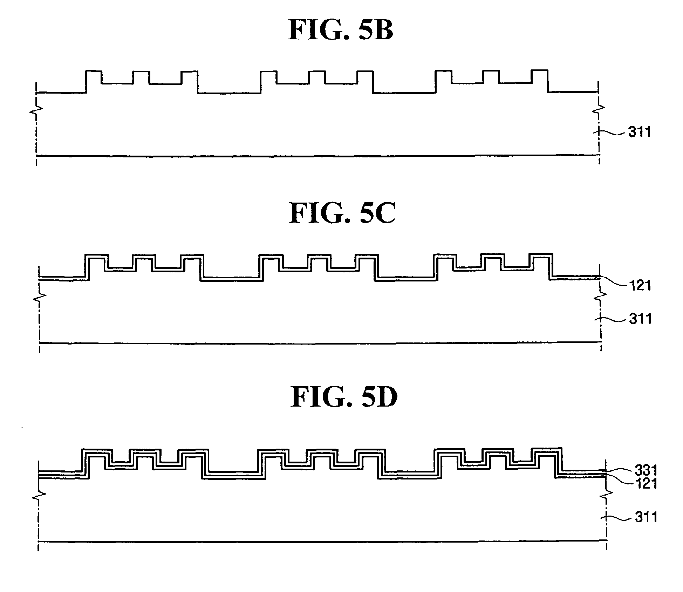

As illustrated in FIG. 5a, a substrate 310 may be provided (S10). The substrate 310 may minimize or reduce the unwanted nonspecific bonds during a hybridization process, and further make the number of nonspecific bonds substantially about zero. The substrate may be made of materials which are transparent to visible ray and/or UV. The substrate 310 may be a flexible and/or rigid substrate. Examples of the flexible substrate may include membranes or plastic films made of nylon and/or nitrocellulose. Examples of the rigid substrate may include a silicon substrate and/or a transparent glass substrate of soda-lime glass. In the case of the silicon substrate and/or the transparent glass substrate, nonspecific bonding may hardly occur during a hybridization process. Additionally, in the case of a transparent glass substrate, it is transparent to visible ray and/or UV, thereby advantageously detecting fluorescent markers. The silicon substrate and/or the transparent glass substrate may be advantageous because the various processes producing thin films or photolithography process typically used to produce semiconductor devices or LCD panels may be applied to them without modification. As illustrated in FIG. 5b, a three-dimensional surface may be formed on the surface of the substrate 310 (S20).

Although not shown in the drawings, a polymer layer may be formed for forming the three-dimensional surface on the substrate 310. Examples of the polymer layer for forming the three-dimensional surface of the substrate 310 may include a silicon oxide film, e.g., a PE-TEOS film, an HDP oxide film, a P-SiH₄ oxide film, and a thermal oxide film, silicates, e.g., hafnium silicates and zirconium silicates, a metal oxynitride film, e.g., a silicon oxynitride film, and a zirconium oxynitride film, a metal oxide film, e.g., a titanium oxide film, a tantalum oxide film, an aluminum oxide film, a hafnium oxide film, a zirconium oxide film, and ITO, polyimides, polyamines, metal, e.g., gold, silver, copper, and palladium, or polystyrenes, polyacrylic acids, and polyvinyls. A deposition process, which is stable during a process of producing semiconductors or a process of producing LCDs for the polymer layer for forming the three-dimensional surface, for example, CVD (Chemical Vapor Deposition), SACVD (Sub-Atmospheric Chemical Vapor Deposition), LPCVD (Low Pressure Chemical Vapor Deposition), PECVD (Plasma Enhanced Chemical Vapor Deposition), sputtering and/or spin coating, may be used. A substance, which is capable of being stably formed on the substrate 310, may be used. After a photoresist film is formed, the photoresist film may be exposed in a projection exposing device by using a photomask, which is prepared according to the three-dimensional patterns to be formed, thereby obtaining the three-dimensional surface of the substrate. The three-dimensional pattern formed on the surface of the substrate may be, for example, a checkerboard pattern.

Thereafter, as shown in FIG. 5c, an immobilization layer 121 may be formed on the substrate 311 having the three-dimensional surface (S30). The immobilization layer 121 may be, for example, a siloxane resin layer made of a siloxane resin, for example, a layer including Si(OR)₃ (wherein R is alkyl). By interposing the immobilization layer 121, the intermediate film 331 (see FIG. 5d) may be more easily coupled with the substrate 311, thereby increasing the reaction yield with the target sample, as compared with the case where the immobilization layer 121 is not interposed. If the surface of the substrate 311 has, for example, a hydroxyl group, the SiO(OR)₃ group of the immobilization layer 121 may be coupled, thereby forming an immobilization layer 121 on the surface of the substrate 311.

For example, the preparation method in which a siloxane resin layer, for example, a siloxane resin layer including -Si(OR)₃ as the immobilization layer 121, will be described. The siloxane resin layer may be formed by, for example, a slit coating process and/or a spin coating process. The slit coating and/or spin coating process may be simpler and more convenient than an oxidation process or chemical vapor deposition process, and the process time may be less, thereby increasing the process yield. The resulting siloxane resin layer may be baked. The baking temperature may be in the range of about 100°C to about 400°C, e.g., about 200°C to about 300°C. The baking time may be in the range of about 30 seconds to about 1 hour. As a result of the baking, the siloxane resins may be crosslinked with each other, which increases rigidity of the siloxane resin layer.

Thereafter, as shown in FIG. 5d, an intermediate film 331 including the chemical structure represented by Formula 1 may be formed on the immobilization layer 121 (S40). Chemicals for forming the intermediate film 331 including the chemical structure represented by Formula 1 may be formed in the following method. 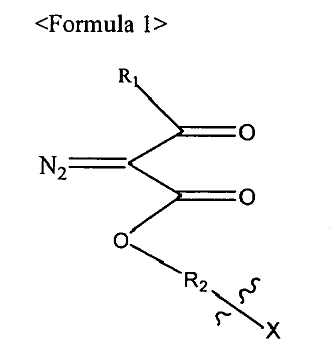
(wherein R₁ is alkyl, aryl, alkoxy, nitrile, ester, phenyl, hydroxyl, aliphatic lactone, cycloalkyl or cycloalkenyl,
R₂ is alkyl, aryl, alkoxy, nitrile, ester, phenyl, hydroxyl, aliphatic lactone, cycloalkyl or cycloalkenyl, and
X is a site coupled with the substrate directly or via an immobilization layer).

The chemicals for forming the intermediate film 331 including the chemical structure represented by Formula 1 may include a diazoketo group. A method for preparing a compound containing a diazoketo group will be described. The diazoketo group may be formed, for example, by subjecting a dienophile compound and a conjugated diene compound to a Diels-Alder reaction for cyclization to synthesize diketones containing a diketo structure, and reacting the diketone with a diazo transfer reagent, e.g., p-carboxybenzenesulfonyl azide, p-toluene -sulfonyl azide, and p-dodecylbenezene sulfonyl azide, at about 0 °C under atmospheric pressure for about 30 minutes to about 60 minutes.

The Diels-Alder Reaction(Diels, O., Alder, K. (1928) "Synthesen in der hydroaromatischen Reihe" Liebigs Annalen der Chemie 460 (1), 98-122) is a reaction for preparing a six-membered ring compound by a 1,4-addition reaction of dienophile having a double bond or triple bond to a conjugated diene. The mechanism of the Diels-Alder Reaction has not been clarified, but the reaction itself may be relatively fast and may or may not require a catalyst. In many cases, by simply mixing dienophile and diene, the reaction may be completed.

For example, in the case of forming the diazoketo group contained in the intermediate film 331, a diene group selected from the compounds represented by: 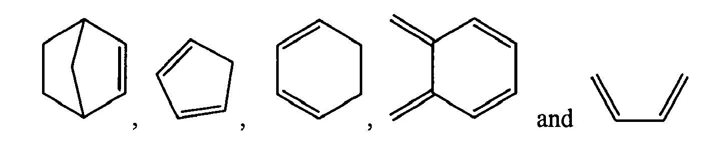 and dienophile selected from the compounds represented by: 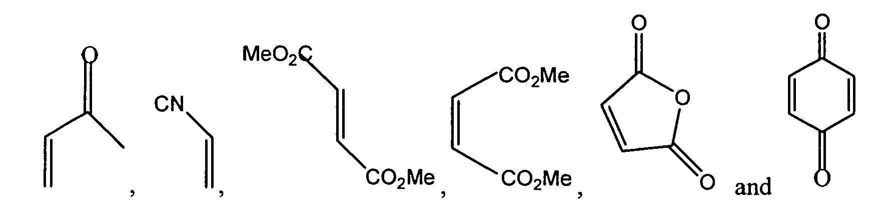 may be subject to a Diels-Alder reaction, to form a six-membered ring compound. If one compound is diene, the other compound may be dienophile.

For example, as shown in the following Scheme 2, cyclopentadiene may be added to a methyl vinyl ketone solution, and 2-acetyl-5-norbornene may be prepared by a Diels-Alder reaction. The resultant may be reacted with dimethyl carbonate to prepare methyl (5-norbornenyl)-3-oxo-propionate.

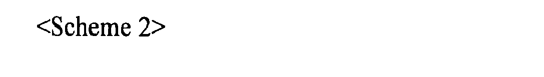

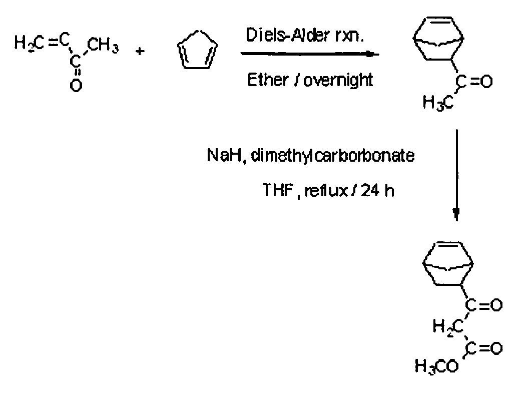

The intermediate film 331 formed as a compound containing a diazoketo group may be coupled with an immobilization layer 121 on the substrate 311. The coupling may be a covalent bond among chemical bonds. By the reaction of the immobilization layer 121 and various functional groups on the surface of the intermediate film 331, the intermediate film 331 and the immobilization layer 121 may be coupled. For example, the intermediate film 331 and the immobilization layer 121 may be coupled with dienophile and diene by a Diels-Alder reaction.

Then, methyl (5-norbornenyl)-2-diazo-oxo-propinate can be manufactured using triethylamine and p-carboxybenzenesulfonyl azide, which is a diazo transfer reagent, after mixing acetonitrile with methyl (5-norbornenyl)-3-oxo-propionate and cooling the mixture, which is shown in the following scheme 3.

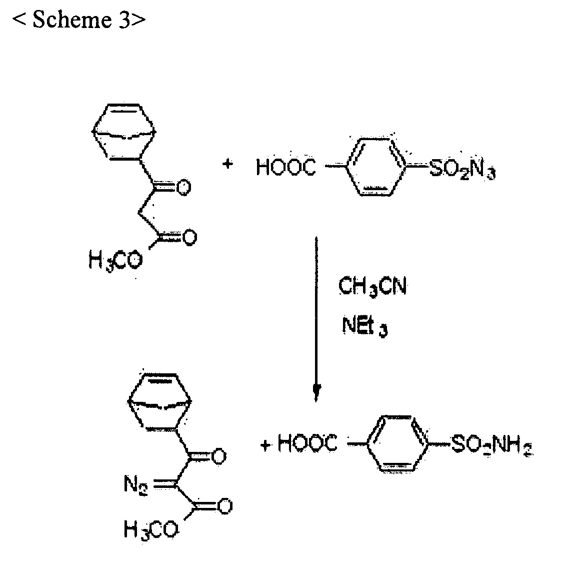

For example, with reference to the chemical structure represented by the following Formula 3, alkene as a functional group of triethoxy(10-undecenyl)-silane formed as the immobilization layer 121 on the substrate 311 may be a diene group. The 2-(13-hydroxy-2-oxtridecanyl)-purane of the intermediate film 331 containing a diazoketo group may be a dienophile group. The diene group of the immobilization layer 121 and the dienophile group of the intermediate film 331 may be coupled by a Diels-Alder reaction.

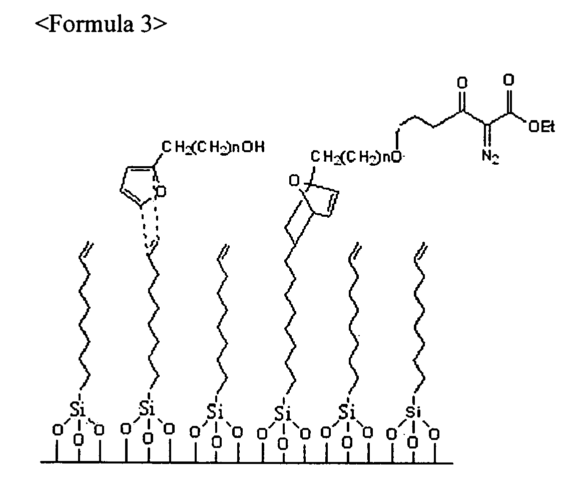

In addition to the Diels-Alder reaction, the immobilization layer 121 and the intermediate film 331 may be coupled through various functional groups. Even when any one of the immobilization layer 121 and the intermediate film 331 contains a functional group, e.g., amine, a carboxylic group, and a hydroxyl group, and the other is a compound which reacts with the functional group, the immobilization layer 121 and the intermediate film 331 may be coupled.

In FIG. 4, and FIGS. 5c to 5d, a method in which the immobilization layer 121 and the intermediate film 331 may be formed is exemplified, but may not be limited thereto. The immobilization layer 121 and the intermediate film 331 may be previously formed in a separate process, and then they may be coupled on the substrate 331. For example, the intermediate film 331 and the immobilization layer 121 may be previously coupled to form the compound represented by the following Formula 4, and then the formed compound may be coupled with the substrate 331. 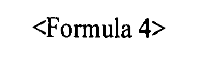 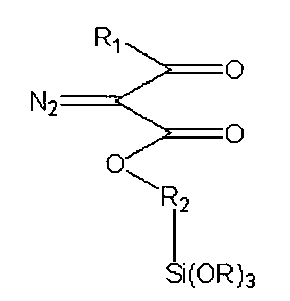
(wherein R₁ is alkyl, aryl, alkoxy, nitrile, ester, phenyl, hydroxyl, aliphatic lactone, cycloalkyl or cycloalkenyl,
R₂ is alkyl, aryl, alkoxy, nitrile, ester, phenyl, hydroxyl, aliphatic lactone, cycloalkyl or cycloalkenyl, and
R₃ is alkyl).

If the substrate 331 is, for example, a hydroxyl group, the Si(OR₃)₃ group of the compound represented by Formula 4 coupled with the intermediate film 331 and the immobilization layer 121 may be coupled with the hydroxyl group of the substrate. The formed intermediate film 331 may provide free interaction, e.g., hybridization, of the oligomer probe with a target sample as a linker or a spacer in the oligomer probe array.

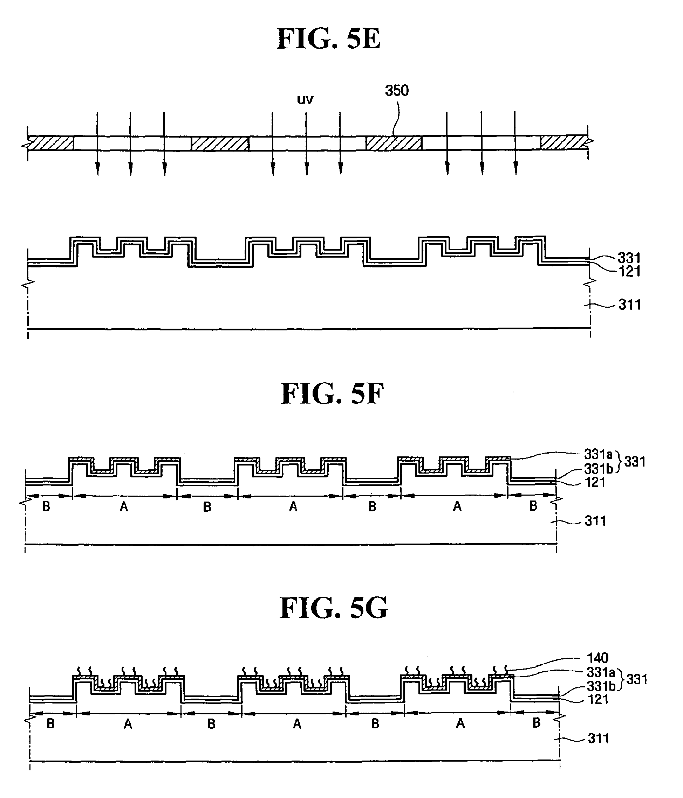

As illustrated in FIG. 5e and FIG. 5f, at least a portion of the formed intermediate film 331 may be exposed to form an intermediate film 331a with a region having the carboxylic group exposed, and an intermediate film 331b including the chemical structure of Formula 1 (S50). In order to produce the oligomer probe array 303 according to example embodiments as illustrated in FIG. 3d, a portion of the formed intermediate film 331 may be exposed. If a portion of the formed intermediate film 331 is exposed, only specific regions of the intermediate film 331 may be selectively coupled with the oligomer probe. The partially exposed region may be an intermediate film 331a corresponding to the activated region A which is capable of coupling with the oligomer probe 160 in the intermediate film 331, and the unexposed region may be an intermediate film 331b corresponding to the deactivated region B which is not capable of coupling with the oligomer probe 160.

Exposure of at least a portion of the intermediate film 331 may be performed by a light at a wavelength in the range of about 190 nm to about 450 nm. Light for exposing a photo-labile protective group, which is to be used for coupling of the oligomer probe, may be more than about 340 nm. In S50, apart from the light used when the photo-labile protective group is used, a light at about 248 nm, may also be used, thereby possibly increasing resolution and assisting in relatively high integration of the oligomer probe array. Here, the wavelength area that exposes the intermediate film 331 may be made different from the wavelength area that deblocks the photolabile protector so that the intermediate film 331 is not damaged in the process of deblocking the protector. However, the wavelength area that exposes the intermediate film 331 may not be different from the wavelength area that deblocks the photolabile protector depending on the manufacturing method and processes of the oligomer probe array.

The chemical structure represented by Formula 1 may contain a diazoketo group. If a ray of about 193 nm or about 248 nm is irradiated on the intermediate film 331 containing a diazoketo group, the diazo group may leave to form a carboxylic acid by a series of reactions as shown in the following Scheme 1.

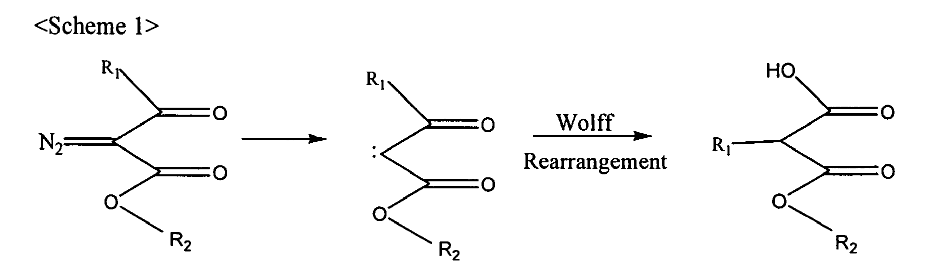

If the intermediate film 331 is partially exposed, the intermediate film 331 may separate the intermediate film 331a including the exposed carboxylic group by exposure and the intermediate film 331b including the chemical structure represented by Formula 1. The intermediate film 331a containing a carboxylic group may correspond to an activated region A of the substrate 311, and the intermediate film 331b including the chemical structure represented by Formula 1 may correspond to a deactivated region B of the substrate 311.

Although not shown in the drawings, the carboxylic group of the intermediate film 331a may have a protective group attached thereto. The protective group may refer to a group that prevents or retards attachment sites from participating in a chemical reaction, and deprotecting means that the protective groups are separated from the attachment sites so that the sites participate in the chemical reaction. For example, an acid labile or photo labile protective group may be attached to the carboxylic group bonded with the intermediate film 331 to protect the functional group, and may then be removed prior to coupling with a linker, or monomers for in-situ photolithographic synthesis of an oligomer probe, or coupling of the synthesized oligomer probe 160, thereby exposing the functional group.

As illustrated in FIG. 5g, a linker 140 may be coupled with the intermediate film 331a corresponding to the activated region A (S60). The linker 140, coupled with the intermediate film 331a containing the carboxylic group, may have a functional group capable of reacting with a carboxylic group. By coupling the linker 140 with the carboxylic group of the intermediate film 331a, the intermediate film 331a corresponding to the activated region A of the substrate 311 may have the chemical structure represented by the following Formula 2. 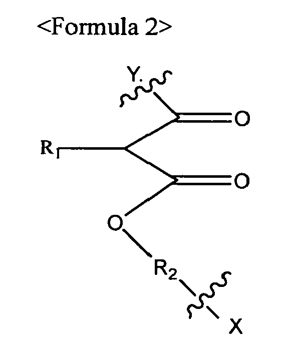
(wherein R₁ is alkyl, aryl, alkoxy, nitrile, ester, phenyl, hydroxyl, aliphatic lactone, cycloalkyl or cycloalkenyl,
R₂ is alkyl, aryl, alkoxy, nitrile, ester, phenyl, hydroxyl, aliphatic lactone, cycloalkyl or cycloalkenyl,
Y is a site coupled with a linker, and
X is a site coupled with the substrate directly or via an immobilization layer).

The linker 140 is shown in FIG. 5g, but may not be limited thereto, and thus, a spacer, the microparticles, and/or nanoparticles may link the intermediate film 331 and the subsequent oligomer probe 160. The linker 140, the spacer, the microparticles, and/or the nanoparticles may contain a functional group capable of coupling with the substrate or the oligomer probe 160, which may make the coupling of the oligomer probe with a substrate easier, or may provide spatial margin for hybridization with a target sample. As discussed earlier, the intermediate film 231 may function the same as the linker 140, the spacer, the microparticles, and/or the nanoparticles, but may also be coupled with the linker 140, the spacer, the microparticles, and/or the nanoparticles to provide an oligomer probe array having an improved reaction yield. The linker 140 may contain a functional group capable of coupling with the carboxylic group of the intermediate film 331a corresponding to the activated region A.

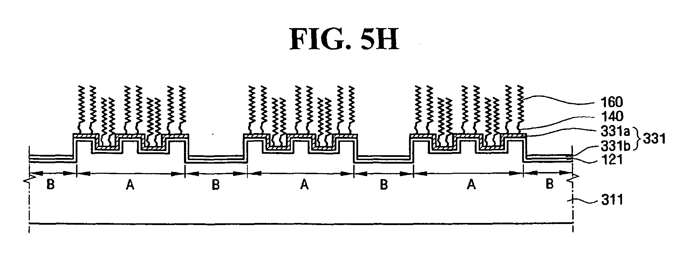

Although not shown in the drawings, a protective group may be attached to effectively couple the linker 140. As mentioned earlier, the protective group may refer to a group that prevents or retards attachment sites from participating in a chemical reaction, and deprotecting means that the protective groups are separated from the attachment sites so that the sites participate in the chemical reaction. Thereafter, as illustrated in FIG. 5h, the oligomer probe 160 may be coupled with the linker 140 (S70). The oligomer probe 160 may be as described for the oligomer probe array 100 according to example embodiments with reference to FIG. 2a, and thus description thereof shall be omitted. The oligomer probe 160 may be coupled with the linker 140, and the coupling may realize a covalent bond among the chemical bonds. Coupling of the oligomer probe 160 with the linker 140 may mean synthesis of the monomers of the oligomer probe 160 by in-situ synthesis. Although not shown in the drawings, a protective group may be attached to effectively couple the monomers of the oligomer probe for the synthesis of the oligomer probe 160. As mentioned earlier, the protective group may refer to a group that prevents or retards attachment sites from participating in a chemical reaction, and deprotecting means that the protective groups are separated from the attachment sites so that the sites participate in the chemical reaction.

As specific examples of the oligomer probe 160, synthesis of an oligonucleotide probe using in-situ photolithography will be described in detail. The functional group of the linker 140 may be exposed, and then a nucleotide phosphoamidite monomer bonded with a photo-labile protective group may be coupled with the exposed functional group. The functional group that does not participate in the coupling may be inactively capped, and oxidation may be performed to convert a phosphite triester structure, which may be formed by bonding between phosphoamidite and 5'-hydroxyl group, into a phosphate structure. As described above, the deprotection of the linker 140 on the activated region A, the coupling of the monomers having the desired sequence, the inactive capping of the functional group which does not participate in the coupling, and the oxidation to achieve conversion into the phosphate structure may be sequentially repeated to synthesize oligonucleotide probes 160 having the desired sequence on each activated region A.

Based on the description of the oligomer probe array 303 according to example embodiments, with reference to FIGS. 1a to 3d, the methods for producing the substrate structure, and the oligomer probe array according to example embodiments will be described. The same reference numerals denote the same elements in the figures of the embodiments, and thus a detailed description of such elements is omitted.

The method for producing the substrate structure according to example embodiments, and the method for producing the oligomer probe according to example embodiments, may include that at least one step in the method for producing the oligomer probe according to example embodiments illustrated in FIGS. 5a-5h is omitted. Although each of the elements is described, the common elements in the description of the oligomer probe array 303 according to example embodiments illustrated in FIG. 3d will be omitted.

The method for producing the substrate structure 100 according to example embodiments, as illustrated in FIG. 1a, may include providing a substrate 110 (S10), and forming an intermediate film 130 including the chemical structure represented by Formula 1 on the formed substrate 110. The method for producing the substrate structure 101 according to example embodiments, as illustrated in FIG. 1b, may include providing a substrate 110 (S10), providing an immobilization layer 120 on the formed substrate 110 (S30), and forming an intermediate film 130 including the chemical structure represented by Formula 1 on the formed immobilization layer 120 (S40).

The method for producing the substrate structure 102 according to example embodiments, as illustrated in FIG. 1c, may include providing a substrate 110 (S10), forming a three-dimensional surface of the substrate 110 (S20), forming an immobilization layer 121 on the three-dimensional surface of the formed substrate 111 (S30), and forming an intermediate film 131 including the chemical structure represented by Formula 1 on the immobilization layer 121 (S40).

The method for producing the oligomer probe array 200 according to example embodiments, as illustrated in FIG. 2a, may include providing a substrate 110 (S10), providing a chemical layer for forming an intermediate film including the chemical structure represented by Formula 1 on the substrate 110 (S40), exposing the entire surface of the chemical layer for forming an intermediate film, thereby exposing a carboxylic group (S50), and coupling the exposed carboxylic group of the intermediate film 230 with the oligomer probe 160 (S70). The entire surface of the intermediate film 230 may be exposed, and the oligomer probe 160 may contain a functional group capable of coupling with a carboxylic group of the intermediate film. As a result, the intermediate film 230 of the oligomer probe array 200 may include the structure represented by the following Formula 2. 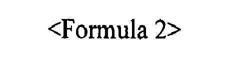 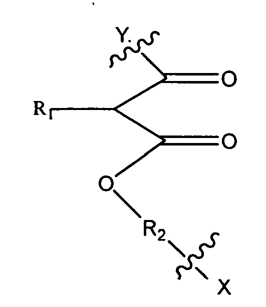
(wherein R1 is alkyl, aryl, alkoxy, nitrile, ester, phenyl, hydroxyl, aliphatic lactone, cycloalkyl or cycloalkenyl,
R2 is alkyl, aryl, alkoxy, nitrile, ester, phenyl, hydroxyl, aliphatic lactone, cycloalkyl or cycloalkenyl,
Y is a site coupled with an oligomer probe, and
X is a site coupled with the substrate directly or via an immobilization layer).

The intermediate film 230 may function as a linker and/or a spacer, which makes the coupling with the oligomer probe 160 and hybridization of a target sample easier. The method for producing the substrate structure 201 according to example embodiments, as illustrated in FIG. 2b, may include providing a substrate 110 (S 10), forming an immobilization layer 120 on the substrate 110(S30), forming an intermediate film 230 including the chemical structure represented by Formula 1 on the immobilization layer 120 (S40), exposing the entire surface of the intermediate film 230, thereby exposing a carboxylic acid (S50), and coupling the exposed intermediate film 230 with the oligomer probe 160 (S70).

The method for producing the substrate structure 202 according to example embodiments, as illustrated in FIG. 2c, may include providing a substrate 110 (S10), forming a three-dimensional surface of the substrate 110 (S20), forming an immobilization layer 121 on the substrate 111 on the three-dimensional surface (S30), forming an intermediate film 231 including the chemical structure represented by Formula 1 on the immobilization layer 121 (S40), exposing the entire surface of the intermediate film 231, thereby exposing a carboxylic group (S50), and coupling the exposed carboxylic group with the oligomer probe 160 (S70).

The method for producing the substrate structure 203 according to example embodiments, as illustrated in FIG. 2d, may include providing a substrate 110 (S10), forming a three-dimensional surface of the substrate 110 (S20), forming an immobilization layer 121 on the substrate having the three-dimensional surface 111 (S30), forming an intermediate film 231 including the chemical structure represented by Formula 1 on the immobilization layer 121 (S40), exposing the entire surface of the intermediate film 231, thereby exposing a carboxylic group (S50), coupling the exposed carboxylic group with the linker 140 (S60), and coupling the linker 140 with the oligomer probe 160 (S70). The linker may have a functional group capable of reacting with a carboxylic group on the intermediate film 231.

The method for producing for the oligomer probe array 300 according to example embodiments, as illustrated in FIG. 3a, may include providing a substrate 310 (S 10), forming a chemical layer used in forming an intermediate film including the chemical structure represented by Formula 1 on the substrate 310 (S40), exposing a portion of the chemical layer for forming an intermediate film to separate the intermediate film 330a having the exposed carboxylic acid and the intermediate film 330b including the chemical structure represented by Formula 1 (S50), and coupling the intermediate film 330a having the exposed carboxylic acid with the oligomer probe 160 (S70). The intermediate film 230 may be exposed partially, and the oligomer probe 160 may contain a functional group capable of coupling with a carboxylic group on the intermediate film. The oligomer probe 160 may not be coupled with the entire surface of the intermediate film 330, but a predetermined or given region 330a by partial exposure, thereby providing a selective activated region. Thus, various oligomer probes may react with a target sample more delicately. The intermediate film 330 may function as a linker (linker molecule) for linking the substrate 310 and the oligomer probe 160 and/or a spacer for providing a spatial margin required for hybridization with a target sample.

The method for producing the oligomer probe array 301 according to example embodiments, as illustrated in FIG. 3b, may include providing a substrate 310 (S10), providing an immobilization layer 120 on the formed substrate 310 (S30), providing a chemical layer for forming an intermediate film including the chemical structure represented by Formula 1 on the formed immobilization layer 120 (S40), exposing a portion of the chemical layer for forming an intermediate film to separate the intermediate film 330a having the exposed carboxylic acid and the intermediate film 330b including the chemical structure represented by Formula 1 (S50), and coupling the intermediate film 330a having the exposed carboxylic acid with the oligomer probe 160 (S70).

The method for producing the oligomer probe array 302 according to example embodiments, as illustrated in FIG. 3c, may include providing a substrate 310 (S10), forming a three-dimensional surface of the substrate 310 (S20), forming an immobilization layer 121 on the substrate having the three-dimensional surface 311 (S30), forming a chemical layer for forming an intermediate film including the chemical structure represented by Formula 1 on the immobilization layer 121 (S40), exposing a portion of the chemical layer for forming an intermediate film to separate the intermediate film 331a having the carboxylic acid exposed and the intermediate film 331b including the chemical structure represented by Formula 1 intact (S50), and coupling the intermediate film 330a having the carboxylic acid exposed with the oligomer probe 160 (S70).

A better understanding of example embodiments may be obtained in light of the following Experimental examples, and constitutions that are not disclosed herein may be easily understood by those skilled in the art.

### <Experimental example 1: Production of substrate structure having three-dimensional surface >

XP4739 (Rohm&Haas Electronic Materials) as a siloxane resin was coated on a silicon substrate to a thickness of about 90 nm using the spin coating process, and hard-baked at about 250°C for about 60 seconds. I7010 was spin-coated on the substrate to a thickness of about 1.2 mm at about 2000 rpm, and then baked at about 100°C for about 60 seconds. The resultant was treated with projection and exposure to light at a wavelength of about 365 nm in ASML PAS5500 100D equipment, using the dark tone mask of a checkerboard pattern type having an opening size of about 11 mm. The resultant was developed with an aqueous about 2.38% TMAH solution to open the regions of length and width straight lines crossing each other. The substrate was subject to plasma etching under CF₄ atmosphere to remove the siloxane from the developed portions of the photoresist, and then remove the remaining photoresist mask by a thinner strip. The resultant was treated with sulfuric acid to activate a hydroxyl group, and the substrate was spin-coupled with an about 0.1% toluene solution of aminotriethoxylpropylsilane using a TEL Mark 8 Act track at about 50 rpm for about 60 seconds, and then dried for about 14 minutes.

The resultant was baked at about 120°C for about 40 minutes, and then washed with flowing DI water for about 10 minutes, using a sonicator for about 15 minutes, and then with DI water for about 10 minutes. Thereafter, the resultant was washed with acetonitrile for about 60 seconds at the TEL Mark 8 Act track. An about 0.1% EtOH solution of N-(3-triethoxylpropyl)-4-hydroxy butyramie was spin-coupled at about 50 rpm for about 60 seconds, washed with IPA, and then cured at about 110°C for about 10 minutes. Using the TEL Mark 8 Act track, about 10 ml of an acetonitrile solution in which nitrophenylpropyloxycarbonyltetraethyleneglycol-cyanoethylphosphor amidite and tetrazole dissolved at about 1 mM and about 5 mM, respectively, was added to the substrate, and left to stand at normal temperature for about 5 minutes. The resultant was treated with acetonitrile under spinning at about 1000 rpm to remove unreacted materials, and the remaining solvent was removed by spin drying at about 2500 rpm.

### <Experimental example 2: Production of immobilization layer >

PAD-Oxide was grown on the silicone substrate to a thickness of about 1000 Å. BC70 KrF Nega PR was spin-coated on the substrate at about 2000 rpm and baked at about 100°C for about 60 seconds. The resultant was treated with projection and exposure by a light at a wavelength of about 248 nm in ASML exposing equipment with a KrF light source, using the dark tone mask of a checkerboard pattern type having an opening size of about 11 mm. The resultant was developed with an aqueous about 2.38% TMAH solution to open the regions of length and width straight lines crossing each other. The substrate was subject to plasma etching under CF₄ atmosphere to remove the portion developed the photoresist, and then to remove the remaining photoresist mask by a thinner strip, thereby opening the patterned PAD-Oxide region. The resultant was treated with piranha (sulfuric acid:hydrogen peroxide =about 70: about 30) at about 120°C for about 1 hour to activate a hydroxyl group of the PAD-Oxide region where an array would be formed. A silane compound containing a diazoketo group may be reacted with the hydroxyl group of the silicone substrate to form a single film. The silane compound was dissolved in toluene, and then immersed on the surface of the silicone to perform a reaction at about 100°C for about 24 hours to about 48 hours.

### <Experimental example 3: Synthesis of compound for intermediate film containing diazoketo group >

### (1) Synthesis of methyl (5-norbornenyl)-3-oxo-propionate

With reference to the following Scheme 2, cyclopentadiene was added to a solution of methyl vinyl ketone in ethyl ether, and reacted at about room temperature for about 12 hours. The solvent was removed off, and the resultant was distilled off under vacuum to obtain pure 2-acetyl-5-norbomene. Dimethyl carbonate, and sodium hydride were sequentially put into a flask including THF charged with nitrogen. 2-Acetyl-5-norbornene was slowly added under stirring. The mixture was reacted at about 80°C for about 24 hours, and then acidified with acetic acid. The solvent was removed, and the residue was then extracted using water and ethyl ether. The extracted organic layer was washed with a sodium chloride solution, and dried over MgSO₄, and then the solvent was removed. The resultant was distilled off under vacuum to obtain pure methyl (5-norbornenyl)-3-oxo-propionate. 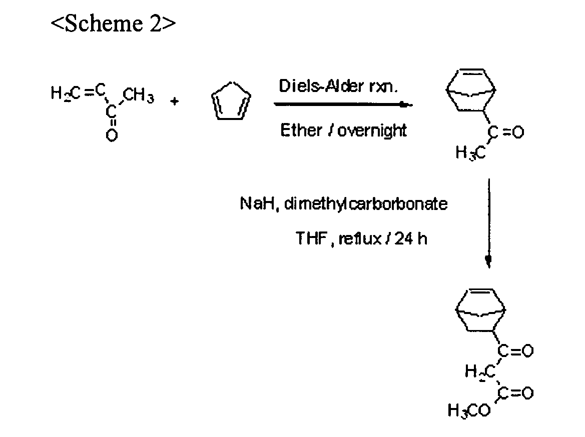 1H NMR
(CDCl3, ppm) : 5.82-6.14 (2H, endo-olefinic proton, exo-olefinic proton), 3.7 (3H, -OCH3), 3.15-3.53 (2H, α-hydrogens), 2.9-3.0 (2H), 1.24-1.9 (5H). 13C NMR (CDC13, ppm) : 204 (-C=O, ketone), 167 (-COO-, ester), 131-138 (C=C), 27.62-52.27 (aliphatic carbons). FT-IR (NaCl plate, cm-1): 1755 (C=O, ketone), 1712 (-COO-, ester), 1625 (C=C, vinyl).

### (2) Synthesis of Methyl (5-norbornenyl)-2-diazo-3-oxo-propionate

With reference to the following Scheme 3, acetonitrile was charged into a flask, methyl (5-norbornenyl)-3-oxo-propionate was added thereto, and cooled with iced water at about 0°C. Triethylamine was slowly added thereto, and p-carboxybenzenesulfonyl azide as a diazo transfer reagent was added to the mixture. The mixture was maintained at about 0°C for about 15 minutes, and reaction was performed at about room temperature for about 2 hours. The solvent was removed, and the residue was extracted using water and petroleum ether. The extracted petroleum ether layer was filtered with a glass filter, the solvent was removed, and the residue was dried under vacuum to obtain methyl (5-norbornenyl)-2-diazo-3-oxo-propionate. 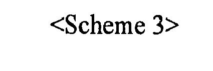 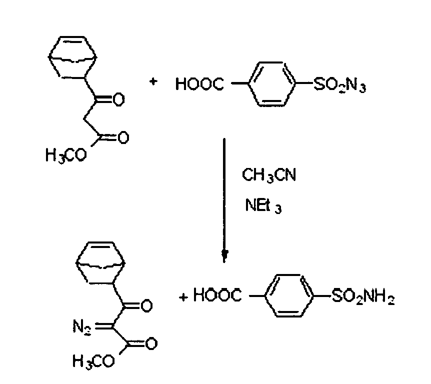 1H NMR (CDCl3, ppm) : 5.82-6.20 (2H, endo-olefinic proton, exo-olefinic proton), 3.7 (3H, -OCH3), 2.9-3.0 (2H), 1.24-1.9 (5H). 13C NMR (CDCl3, ppm) : 194 (-C=O, ketone), 161 (-COO-, ester), 131-138 (C=C), 27.62-52.27 (aliphatic carbons). FT-IR (NaCl plate, cm-1) : 2139 (-N2, diazo),1722 (C=O, ketone), 1651 (-COO-, ester).

### <Experimental example 4: Formation of intermediate film on immobilization layer >

Triethoxy10-undecenyl-silane as an immobilization layer was formed on a substrate. The hydroxyl group of 2-(13-hydroxy-2-oxtridecanyl)-purane was reacted with 2-diazo-3-oxo-hexanolic acid ester methane containing a diazoketo group to form an intermediate film. Alkene of the immobilization layer as the diene group and 2-(13-hydroxy-2-oxtridecanyl)-purane as the dienophile were subject to a Diels-Alder reaction, to form an intermediate film on the immobilization layer, as follows.

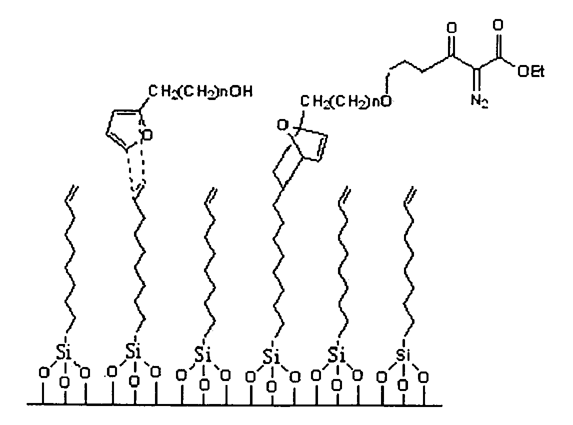

### <Experimental example 5: Formation of spacer>

In Experimental example 4, the formed intermediate film was exposed selectively using an ASML PAS5500 100D stepper, thereby forming a spacer on the intermediate film having the exposed carboxylic group. Using a TEL Mark 8 Act track for elongation of the spacer, about 10 ml of a acetonitrile solution in which nitrophenylpropyloxycarbonyltetraethyleneglycol-cyanoethylphosphor amidite and tetrazole dissolved at about 1 mM and about 5 mM, respectively, was added to the substrate, and left to stand at normal temperature for about 5 minutes. The resultant was spun at about 1000 rpm to remove unreacted materials, and the remaining solvent was removed by spin drying at about 2500 rpm. The residue was washed to have the substrate wet with acetonitrile.

### <Experimental example 6: Growth of nucleic acid>

A photo-reaction was performed on the region in which a nucleic acid reaction would occur using an ASML PAS5500 100D stepper at a wavelength of about 248 nm or about 365 nm, and then subsequent reactions were performed. Using a TEL Mark 8 Act track, the substrate was treated with about 10 ml of acetonitrile solution in which adenine/thymine/guanie/cytosine sugar-phosphoramidite and tetrazole dissolved at about 1 mM and about 5 mM, respectively, and left to stand at normal temperature for about 5 minutes. The resultant was treated with acetonitrile under spinning at about 1000 rpm to remove unreacted materials, and the remaining solvent was removed by spin drying at about 2500 rpm. Using a TEL Mark 8 Act track, the substrate was treated with a solution of Ac2PO/py/methylimidazole (1:1:1) in THF and an about 0.02 M of an iodine solution in THF, to perform capping and oxidation of the nucleic acids uncoupled. Thereafter, nucleic acid synthesis started by light activation. Exposure was performed at an ASML PAS5500 100D stepper at a wavelength of about 365 nm using a checkerboard pattern chrome on a quartz mask having an opening size of about 11 um with an energy of about 5000 mJ, thereby removing the nitroaromatic protective groups. By using adenine/thymine/guanie/cytosine sugar-phosphoramidite, coupling, capping, oxidation, and photo deprotection were repeated, thereby synthesizing a nucleic acid having a desired sequence.

As described above, the substrate structure, the oligomer probe array, and the method for producing the same according to example embodiments may improve integration of the oligomer probe array and the reaction yield. The intermediate film in the substrate structure and the oligomer probe array may function as a linker or a spacer, because the intermediate film may provide a region which selectively reacts with the oligomer probe. The intermediate film may have a lower degree of entanglement, and the reactive group may be allowed to react with a carboxylic group at about 248 nm, thereby giving remarkably higher decomposition.

Although example embodiments have been described in connection with the example embodiments, it will be apparent to those skilled in the art that various modifications and changes may be made thereto without departing from the scope and spirit of example embodiments. Therefore, it should be understood that the above embodiments are not limitative, but illustrative in all aspects.

## Claims

1. A substrate structure comprising:
- a substrate and
- an intermediate film on the substrate, the intermediate film comprising a compound with the structure represented by Formula 1 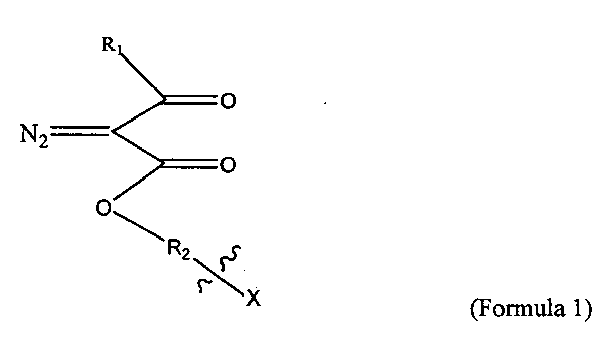 wherein
- R₁ is alkyl, aryl, alkoxy, nitrile, ester, phenyl, hydroxyl, aliphatic lactone, cycloalkyl or cycloalkenyl,
- R₂ is alkyl, aryl, alkoxy, nitrile, ester, phenyl, hydroxyl, aliphatic lactone, cycloalkyl or cycloalkenyl and
- X is coupled with the substrate directly or via an immobilization layer.

2. The substrate structure of claim 1, wherein X is coupled with the substrate via an immobilization layer comprising -Si(OR)₃, wherein R is alkyl.

3. The substrate structure of claim 1, wherein R₁ or R₂ is the product of a Diels-Alder reaction of a compound selected from the group consisting of 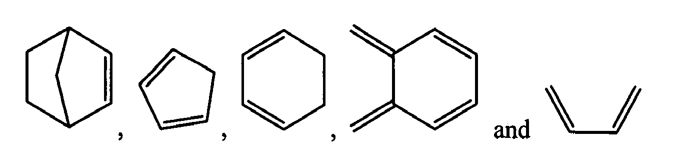 with a compound selected from the group consisting of

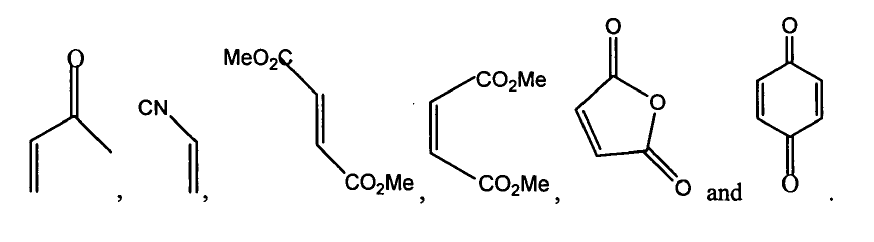

4. The substrate structure of claim 1, wherein the substrate comprises a three-dimensional surface on or in the substrate and the intermediate film is on the three-dimensional surface.

5. An oligomer probe array, comprising:
- a substrate,
- an oligomer probe formed on the substrate and
- an intermediate film interposed between the substrate and the oligomer probe, wherein the intermediate film comprises a compound with the structure represented by Formula 2 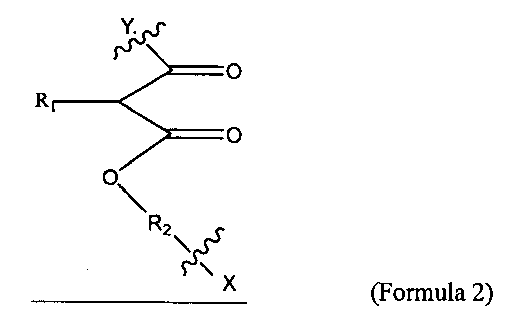 wherein
- R1 is alkyl, aryl, alkoxy, nitrile, ester, phenyl, hydroxyl, aliphatic lactone, cycloalkyl or cycloalkenyl,
- R2 is alkyl, aryl, alkoxy, nitrile, ester, phenyl, hydroxyl, aliphatic lactone, cycloalkyl or cycloalkenyl,
- Y is coupled with a linker, a spacer, or an oligomer probe and
- X is coupled with the substrate directly or via an immobilization layer.

6. The oligomer probe array of claim 5, wherein X is coupled with the substrate via an immobilization layer comprising -Si(OR)₃, wherein R is alkyl.

7. The oligomer probe array of claim 5, wherein R₁ or R₂ is the product of a Diels-Alder reaction of a compound selected from the group consisting of 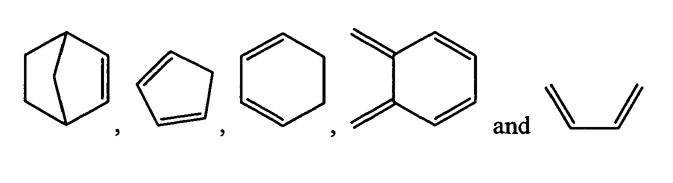 with a compound selected from the group consisting of

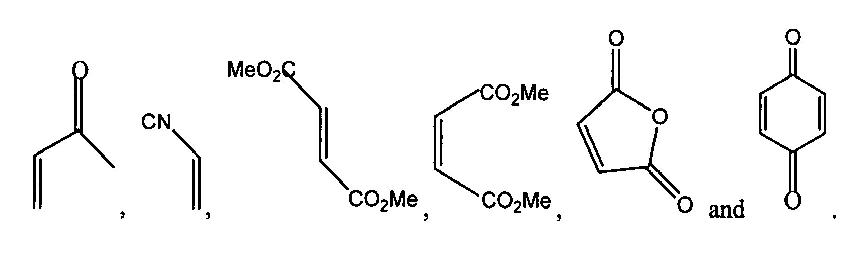

8. The oligomer probe array of claim 5, wherein the substrate comprises a three-dimensional surface on or in the substrate and the intermediate film is on the three-dimensional surface.

9. The oligomer probe array of claim 5, wherein the substrate comprises an activated region coupled with the oligomer probe and a deactivated region not coupled with the oligomer probe, wherein the oligomer probe on the activated region and the substrate are intermediated by the chemical structure represented by Formula 2, and the oligomer probe on the deactivated region and the substrate are intermediated by a chemical structure represented by the following Formula 1.

10. The oligomer probe array of claim 5 or claim 9, further comprising a linker or a spacer between the intermediate film and the oligomer probe on the activated region.

11. A method for producing a substrate structure, comprising:
- providing a substrate and
- forming an intermediate film including a compound with the structure represented by Formula 1 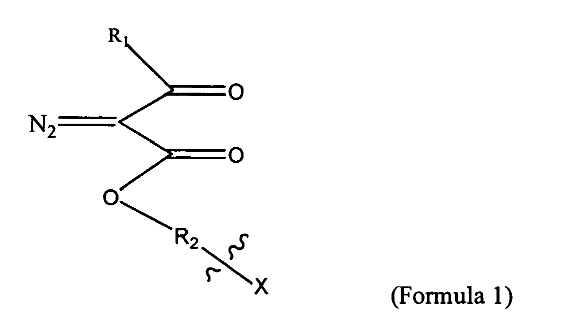 wherein
- R₁ is alkyl, aryl, alkoxy, nitrile, ester, phenyl, hydroxyl, aliphatic lactone, cycloalkyl or cycloalkenyl,
- R₂ is alkyl, aryl, alkoxy, nitrile, ester, phenyl, hydroxyl, aliphatic lactone, cycloalkyl or cycloalkenyl, and
- X is coupled with the substrate directly or via an immobilization layer.

12. The method of claim 11, wherein the immobilization layer comprises -Si(OR)₃, wherein R is alkyl.

13. The method of claim 11, wherein R₁ or R₂ is formed by subjecting a compound selected from the group consisting of 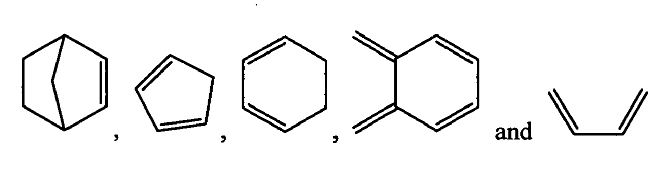 with a compound selected from the group consisting of 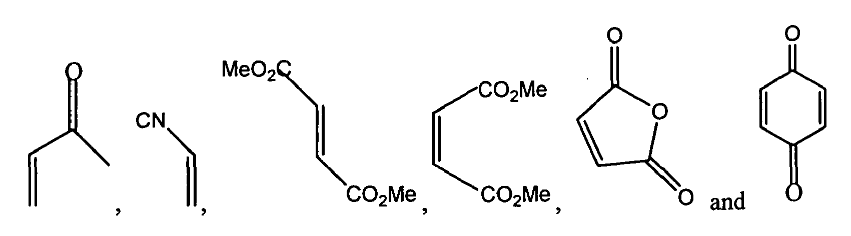 to a Diels-Alder reaction.

14. The method of claim 11, wherein the substrate comprises a three-dimensional surface on or in the substrate, and the intermediate film is formed on the three-dimensional surface.

15. The method of claim 12, wherein the chemical structure represented by Formula 1 is prepared by reacting a compound selected from the group consisting of 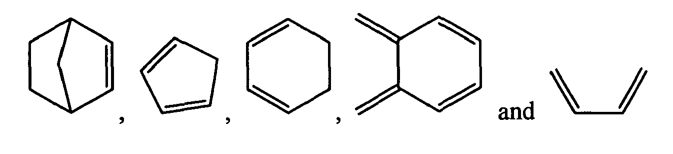 and with a compound selected from the group consisting of 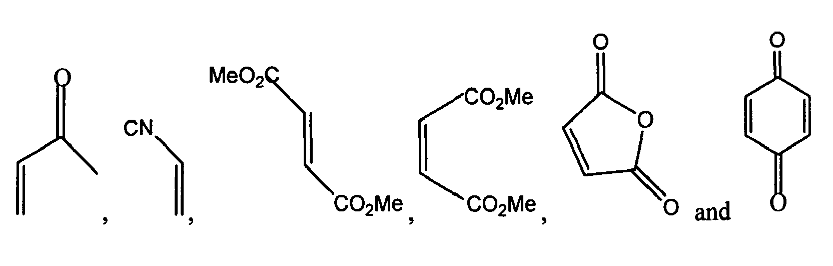 to a Diels-Alder reaction for cyclization to synthesize diketone containing a diketo structure and forming a diazoketo group by reacting the diketone containing the diketo structure with a diazo transfer reagent.

16. The method for producing the substrate structure of claim 15, wherein the diazo transfer reagent is p-carboxybenzenesulfonyl azide, p-toluene -sulfonyl azide, or p-dodecylbenezene sulfonyl azide.

17. A method for producing an oligomer probe array, comprising:
- producing the substrate structure of claim 11,
- exposing at least a portion of the intermediate film and
- coupling at least a portion of the exposed intermediate film with an oligomer probe.

18. The method for producing the oligomer probe array of claim 17, wherein at least a portion of the intermediate film is exposed to light at a wavelength in the range of about 190 nm to about 450 nm.

19. The method for producing the oligomer probe array of claim 17, wherein coupling at least a portion of the exposed intermediate film with the oligomer probe includes coupling via a linker or a spacer.
